# EUROPEAN PATENT APPLICATION

(11) **EP 3 042 682 A1**
(43) Date of publication of application: **13.07.2016**
(21) Application number: 15192831.4
(22) Date of filing: 03.02.2000
(51) Int. Cl.: A61M 16/08

(54) **GAS DELIVERY CONNECTION ASSEMBLY**

(30) Priority: 09.02.1999 AU PP855099
(62) Divisional of application: 05002217.7
(71) Applicant: ResMed Limited, Bella Vista, NSW 2153 (AU)
(72) Inventor: Gunaratnam, Michael Kassipillai, Marsfield 2122 New South Wales (AU); Kwok, Philip Rodney, Chatswood 2067 New South Wales (AU); Lithgow, Perry David, North Ryde 2113 New South Wales (AU)
(74) Representative: Vossius & Partner Patentanwälte Rechtsanwälte mbB

(57) **Abstract**

The present invention relates to a patient gas delivery apparatus including:
a mask adapted for communication with a patient's airways;
a gas flow generator and gas delivery conduit means, further including an assembly connected in series between the conduit and the mask;
said assembly being formed in at least two parts connected by interengaging connecting means, said assembly further including means for connection to the mask, wherein connection of the assembly to the mask prevents incorrect assembly or incorrect alignment of said parts.

## Description

### BACKGROUND OF THE INVENTION

This invention relates to improvements in patient gas delivery apparatus of the kind used in the analysis and treatment of respiratory disorders. The invention will be described with particular reference to patient gas delivery apparatus used in the treatment of respiratory disorders such as Obstructive Sleep Apnea (OSA) but it is not intended to be limited thereto.

Patient gas delivery apparatus of the kind having a mask worn by a patient and a gas delivery conduit attached to the mask, is commonly used in the analysis and treatment of respiratory disorders. The gas conduit delivers a gas under pressure to the patient. It is necessary that the gas conduit is detachable from the mask to facilitate cleaning.

Patient gas delivery apparatus typically includes at a minimum, a gas delivery conduit and a nose or full face mask. In some cases it is a clinical requirement that additional components be included, such as means for CO₂ washout, for example, vents, anti-asphyxia valves and the like. In some cases, these additional components must be assembled in between the gas delivery conduit and the mask. Problems with prior art assemblies include:
(a) They may be inadvertently assembled without the additional components
(b) They may be incorrectly assembled, for example, incorrectly aligned
(c) During the course of treatment, the patient may inadvertently remove or dismantle the assembly and incorrectly reassemble it.

### SUMMARY OF THE INVENTION

The present invention is directed towards solving or ameliorating one or more of these problems. The invention will be described with reference to a full face mask and an anti-asphyxia valve, though other forms of mask and additional components may be used.

In one form, the invention resides in a patient gas delivery apparatus including:
a mask structured to communicate with a patient's airway;
a gas flow generator structured to supply pressurized gas;
a gas delivery conduit connected to the gas flow generator; and
a connecting assembly including an anti-asphyxia valve, the assembly being connected in series between the conduit and the mask and providing a series air flow path between the conduit and the mask in normal operation;
the connecting assembly having a distal end and a proximal end, the distal end being configured and positioned to connect to the conduit, and the proximal end being configured and positioned to connect to the mask;
wherein the mask and the conduit are adapted to prevent direct interconnection without the connecting assembly.

In a further form of the invention, there is provided an assembly adapted for connection in series between a gas delivery conduit and a patient mask in a patient gas delivery apparatus, the connecting assembly comprising:
a first housing having a distal end and a proximal end, the distal end configured and positioned to connect to the conduit and the proximal end configured and positioned to connect to the mask; and
an anti-asphyxia housing forming part of the first housing,
the anti-asphyxia valve housing providing a series air flow path between the conduit and the mask in normal operation;
wherein the mask and the conduit are adapted to prevent direct interconnection without the connecting assembly.an assembly for connection in series between a gas delivery conduit means and a patient mask in a patient gas delivery apparatus, the assembly being formed in at least two parts connected by interengaging connecting means, said assembly further including means for connection to the mask, wherein connection of the assembly to the mask prevents disengagement of the interengaging connecting means such that said at least two parts of the assembly cannot separate whilst the assembly is connected to the mask.

A further form of the invention provides a patient gas delivery apparatus including:
a mask having an aperture in communication with a patient's airway;
a gas flow generator structured to supply pressurized gas;
a gas delivery conduit having first and second ends, the first end being connected to the gas flow generator;
a connecting assembly connected in series between the conduit and the mask, the connecting assembly including a first housing having a rotatable coupling at a distal end and a mating portion at a proximal end, the rotatable coupling being configured and positioned to connect to the second opposing end of the conduit and the mating portion being configured and positioned to be inserted through the aperture of the mask; and an anti-asphyxia valve housing forming part of the first housing,
the anti-asphyxia valve housing providing a series air flow path between the conduit and the mask in normal operation;

In a further form, the invention provides an assembly adapted for connection in series between a gas delivery conduit and a patient mask in a patient gas delivery apparatus, the assembly comprising:
a first part adapted to be connected to one of the conduit and the mask; and
a second part connected to the first portion and adapted to be connected to the other of the conduit and the mask, the second part including an anti-asphyxia valve housing and an anti-asphyxia valve member provided to the housing,
   the anti-asphyxia valve housing providing a series air flow path between the conduit and the mask in normal operation;
wherein the mask and the conduit are adapted to prevent direct interconnection without the anti-asphyxia valve assembly.

In one preferred form of the invention, the assembly may form a housing for one or more internal components, for example a valve member or a flow sensor.

Further preferred embodiments are described by the following aspects:
1. A patient gas delivery apparatus including:
   a mask structured to communicate with a patient's airway;
   a gas flow generator structured to supply pressurized gas;
   a gas delivery conduit connected to the gas flow generator; and
   a connecting assembly including an anti-asphyxia valve, the assembly being connected in series between the conduit and the mask and providing a series air flow path between the conduit and the mask in normal operation;
   the connecting assembly having a distal end and a proximal end, the distal end being configured and positioned to connect to the conduit, and the proximal end being configured and positioned to connect to the mask;
   wherein the mask and the conduit are adapted to prevent direct interconnection without the connecting assembly.
2. A patient gas delivery apparatus according to aspect 1, wherein the assembly includes a housing having at least a first part and a second part connected by a releasable engagement means.
3. A patient gas delivery apparatus according to aspect 2, wherein the first part of the assembly includes a rotatable coupling providing the distal end of the assembly to connect to the conduit.
4. A patient gas delivery apparatus according to aspect 2, wherein the second part of the assembly includes a mating portion providing the proximal end of the assembly to connect to the mask.
5. A patient gas delivery apparatus according to aspect 4, wherein the mating portion is inserted into an aperture provided in the mask and a clip is attached to the mating portion from an inner side of the mask so as to prevent withdrawal of the mating portion from the aperture.
6. A patient gas delivery apparatus according to aspect 2, wherein the housing has at least one vent that is closed by the valve member during normal operation of the said apparatus, and opened when pressure falls below a predetermined pressure.
7. An assembly adapted for connection in series between a gas delivery conduit and a patient mask in a patient gas delivery apparatus, the connecting assembly comprising:
   a first housing having a distal end and a proximal end, the distal end configured and positioned to connect to the conduit and the proximal end configured and positioned to connect to the mask; and
   an anti-asphyxia housing forming part of the first housing,
   the anti-asphyxia valve housing providing a series air flow path between the conduit and the mask in normal operation;
   wherein the mask and the conduit are adapted to prevent direct interconnection without the connecting assembly.
8. A connecting assembly according to aspect 7, wherein the housing has at least a first part and a second part connecting by a releasable engagement means.
9. A connecting assembly according to aspect 8, wherein the first part of the assembly includes a rotatable coupling providing the distal end of the housing to connect to the conduit.
10. A connecting assembly according to aspect 8, wherein the second part of the assembly includes a mating portion providing the proximal end of the housing to connect to the mask.
11. A connecting assembly according to aspect 10, wherein the mating portion is inserted into an aperture provided in the mask and a clip is attached to the mating portion from an inner side of the mask so as to prevent withdrawal of the mating portion from the aperture.
12. A connecting assembly according to aspect 7, wherein the housing has at least one vent that is closed by the valve member during normal operation of the apparatus, and opened when pressure falls below a predetermined pressure.
13. A patient gas delivery apparatus including:
   a mask having an aperture in communication with a patient's airway;
   a gas flow generator structured to supply pressurized gas;
   a gas delivery conduit having first and second ends, the first end being connected to the gas flow generator;
   a connecting assembly connected in series between the conduit and the mask, the connecting assembly including a first housing having a rotatable coupling at a distal end and a mating portion at a proximal end, the rotatable coupling being configured and positioned to connect to the second opposing end of the conduit and the mating portion being configured and positioned to be inserted through the aperture of the mask; and
   an anti-asphyxia valve housing forming part of the first housing,
   the anti-asphyxia valve housing providing a series air flow path between the conduit and the mask in normal operation;
   wherein the mask and the conduit are adapted to prevent direct interconnection without the connecting assembly.
14. A patient gas delivery apparatus according to aspect 1, wherein the mask includes an aperture for receiving pressurized air, the connecting assembly including the anti-asphyxia valve housing positioned upstream of the aperture.
15. A patient gas delivery apparatus according to aspect 1, further comprising a cushion, the cushion structured to fit over the mask.
16. A patient gas delivery apparatus according to aspect 1, wherein the assembly is an elbow joint.
17. A patient gas delivery apparatus according to aspect 1, further comprising a cushion, the cushion being removable from the mask.
18. A patient gas delivery apparatus according to aspect 1, wherein the anti-asphyxia valve is positioned outside of the mask.
19. A connecting gas assembly according to aspect 7, wherein the connecting assembly is an elbow joint.
20. A gas assembly according to aspect 7, wherein the anti-asphyxia valve is positioned outside of the mask.
21. A patient gas delivery apparatus according to aspect 13, wherein the mask includes an aperture for receiving pressurized air, the assembly including the anti-asphyxia valve member being positioned upstream of the aperture.
22. A patient gas delivery apparatus according to aspect 13, further comprising a cushion, the cushion structured to fit over the mask.
23. A patient gas delivery apparatus according to aspect 13, wherein the connecting assembly is an elbow joint.
24. A patient gas delivery apparatus according to aspect 13, further comprising a cushion, the cushion being removable from the mask.
25. A patient gas delivery apparatus according to aspect 13, wherein the anti-asphyxia valve is positioned outside of the mask.
26. An assembly adapted for connection in series between a gas delivery conduit and a patient mask in a patient gas delivery apparatus, the assembly comprising:
   a first part adapted to be connected to one of the conduit and the mask; and
   a second part connected to the first portion and adapted to be connected to the other of the conduit and the mask, the second part including an anti-asphyxia valve housing and an anti-asphyxia valve member provided to the housing,
      the anti-asphyxia valve housing providing a series air flow path between the conduit and the mask in normal operation;
   wherein the mask and the conduit are adapted to prevent direct interconnection without the anti-asphyxia valve assembly.
27. A connecting assembly according to aspect 26, wherein the first part and second part are connected by releasable engagement means.
28. A connecting assembly according to aspect 27, wherein on of the first and second parts includes a rotatable coupling adapted to connect to the conduit.
29. A connecting assembly according to aspect 27, wherein on of the first and second parts includes a mating portion adapted to connect to the mask.
30. A connecting assembly according to aspect 29, wherein the mating portion is inserted into an aperture provided in the mask and a clip is attached to the mating portion from an inner side of the mask so as to prevent withdrawal of the mating portion from the aperture.
31. A connecting assembly according to aspect 26, wherein the anti-asphyxia valve housing has at least one vent that is closed by the valve member during normal operation of the apparatus, and opened when pressure falls below a predetermined pressure.
32. Patient gas delivery apparatus including a mask adapted for communication with a patient's airways, a gas flow generator and gas delivery conduit means, further including an assembly connected in series between the conduit means and the mask, said assembly being formed in at least two parts connected by interengaging connecting means, said assembly further including means for connection to the mask, wherein connection of the assembly to the mask prevents disengagement of the interengaging connecting means such that said at least two parts of the assembly cannot separate whilst the assembly is connected to the mask.
33. Patient gas delivery apparatus according to aspect 31, wherein said interengaging connecting means includes detent means on a first of said parts of the assembly, said detent means releasably engaging a second of said parts of the assembly and being held in engaged position by the mask whilst the assembly is connected to the mask.
34. Patient gas delivery apparatus according to aspect 33, wherein said first and second parts form a housing for a flow sensor of said apparatus.
35. Patient gas delivery apparatus according to aspect 33, wherein said first and second parts form a housing for an anti-asphyxia valve member.
36. Patient gas delivery apparatus according to aspect 35, wherein said housing has at least one vent which is closed by said valve member during normal operation of said apparatus, opening when pressure falls below a predetermined pressure.
37. Patient gas delivery apparatus according to aspect 33, wherein said second part includes said means for connection of the assembly to the mask.
38. Patient gas delivery apparatus according to aspect 37, wherein said means for connection to the mask includes a mating portion for insertion into an aperture of the mask and locking means attachable to said mating portion from an inner side of the mask so as to prevent withdrawal of the mating portion from said aperture, said detent means being prevented from disengagement from said second part whilst said mating portion is inserted in said aperture.
39. Patient gas delivery apparatus according to aspect 38, wherein said detent means is prevented from said disengagement by contact with the mask.
40. Patient gas delivery apparatus according to aspect 39, wherein the detent means is resiliently biased in a radial direction relative to a common axis of said aperture and said mating portion such that the detent means engages behind a respective formation on said second part and wherein opposite radial movement of said detent means to disengage from said second part is prevented by said contact.
41. Patient gas delivery apparatus according to aspect 40, wherein said disengagement is prevented by a projection on the mask.
42. Patient gas delivery apparatus according to aspect 41, wherein said projection includes a projecting rim surrounding said aperture.
43. Patient gas delivery apparatus according to aspect 32, wherein said means for connection of the assembly to the mask includes locking means located on an inner side of said mask, such that substantial removal of the mask from the patient is a prerequisite for disconnection of the assembly from the mask and disengagement of said interengagement means.
44. Patient gas delivery apparatus according to aspect 32, wherein a distal end of the assembly includes rotatable coupling means for connection of the conduit.
45. Patient gas delivery apparatus according to aspect 44, wherein the mask and the conduit are not adapted for direct interconnection without the assembly.
46. An assembly for connection in series between a gas delivery conduit means and a patient mask in a patient gas delivery apparatus, the assembly being formed in at least two parts connected by interengaging connecting means, said assembly further including means for connection to the mask, wherein connection of the assembly to the mask prevents disengagement of the interengaging connecting means such that said at least two parts of the assembly cannot separate whilst the assembly is connected to the mask.
47. An assembly according to aspect 46, further including an anti-asphyxia valve member housed in said assembly.
48. An assembly according to aspect 47, wherein said assembly has at least one vent, said valve member being adapted to close said vent during normal operation of the apparatus and to open when pressure falls below a predetermined pressure.

Further preferred embodiments of the invention will now be described by way of example only with reference to the accompanying drawings in which:
Figure 1 is a perspective view showing the mask, anti-asphyxia valve housing and conduit connection assembly;
Figure 2 is an exploded view of the anti-asphyxia valve and conduit connection assembly; and
Figure 3 is an exploded view of the mask assembly.

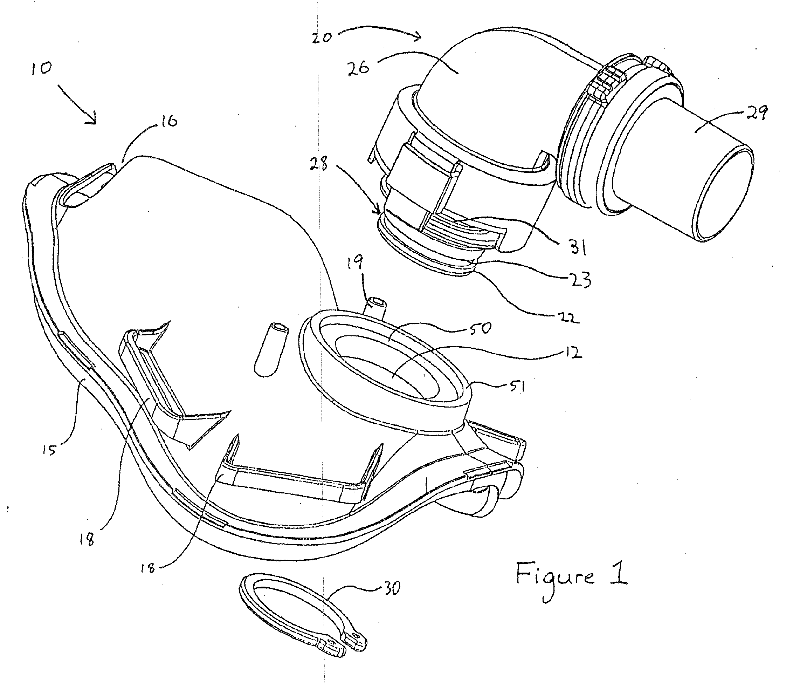

In Figure 1 a mask frame is shown generally at 10. The mask is designed to be worn on a patient's face and is secured by means of straps (not shown) received by attachment points 18.

A conduit end assembly is shown generally at 20, including an elbow part 26 having at one end thereof a combined vent/connector piece 28. The elbow and vent/connector piece together form a housing for an anti-asphyxia valve or other internal components (not shown). At the other end of the elbow is a detachable swivel tube 29 for connection of the gas delivery conduit (not shown).

The mask 10 includes a circular aperture 12 sized to receive a mating portion 22 of the vent/connector piece 28. The mating portion 22 has an annular groove 23 formed therein that receives a locking means 30 in the form of a C-shaped clip attached after mating to the mask. The clip 30 has an outside diameter greater than the width of the aperture 12 and an inner diameter adapted to ensure a snug fit within the annular groove 23. The clip 30 is resilient and can expand sufficiently to allow the clip to be fitted into and removed from the groove 23. As shown in Figure 1, the clip 30 is located onto the mating portion 22 on the inside of the mask 10. In this position, the clip 30 is inaccessible while the mask is being worn by a patient. Once the mating portion 22 of the vent/connector piece 28 has been inserted through the aperture 12 and the locking clip placed in the annular groove, the conduit end assembly 20 and the mask 10 cannot be separated without first removing the mask from the patient.

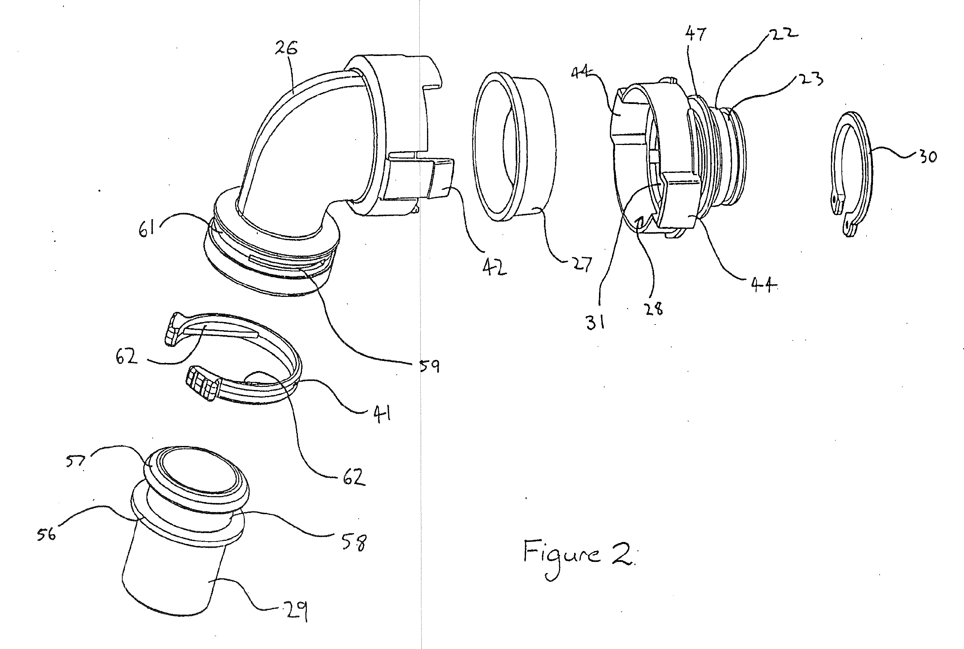

An exploded view of the anti-asphyxia valve and conduit connector assembly is shown in Figure 2.

The end of the elbow 26 adjacent the mask 10 is fitted with an anti-asphyxia valve arrangement that provides an air passage to the patient in the event of failure of the gas delivery apparatus, consisting of a valve membrane 27 fitted into the end of elbow 26 and vents 31 in the vent/connector piece 28. During proper operation of the gas delivery system, the valve membrane remains in the orientation shown in Fig. 2, closing off the vents 31. In the event of a drop in pressure below a predetermined level, the valve membrane 27 flips to a reverse orientation, opening the vents 31. The construction and operation of the anti-asphyxia valve is described in more detail in the Applicant's Australian Patent Application No. 65527/99, the contents of which are incorporated herein by reference.

Resilient detents 42 on the elbow 26 pass through and engage behind slot-forming formations 44 in the vent/connector piece 28 to provide releasable engagement of the two parts.

The vent/connector piece has a collar 47 that abuts a corresponding surface of the mask 10 to limit the distance that the vent/connector piece can be inserted into the mask aperture 12 (Figure 1). The corresponding surface is an annulus 50 having a protruding rim 51 the outer circumference of which preferably engages the inner surface of the detents 42 on insertion of the mating portion 22 into the aperture 12. This engagement prevents the detents from being pushed radially inwards sufficiently for the detents to disengage from behind the slot-forming formations 44, thus preventing the elbow 26 and vent/connector piece 28 from separating whilst still attached to the mask frame 11, for example during patient treatment. The result of this is that the anti-asphyxia valve arrangement cannot be disassembled without first removing the elbow and vent/connector piece assembly from the mask. However, once disconnected from the mask, the assembly may be readily separated for cleaning and then reassembled.

The other, distal end of elbow 26 has an enlarged diameter portion which receives the swivel tube 29, onto which a flexible gas conduit (not shown) may be fitted. The swivel tube 29 has a pair of flanges 56 and 57 defining an annular groove 58 therebetween. The end of swivel tube 29 is inserted into the elbow 26 until the end flange 57 abuts an inner surface (not shown) within elbow 26. In this position the annular groove 58 is at least partially aligned with an annular groove 61 in the exterior of the elbow, which receives a swivel clip 41.

The swivel clip 41 has an inner diameter only slightly greater than the diameter of the groove 61, to ensure a snug fit within the groove. The clip 41 is resilient to permit sufficient expansion for attachment and removal of the clip from the groove. The groove 61 has slots 59 which receive lugs 62 on the clip. These lugs rotatably engage in the groove 58 between flanges 56 and 57 of the swivel tube. The swivel tube arrangement thus acts as a rotatable coupling between the conduit and the elbow whilst allowing quick attachment and removal of the gas conduit from the elbow regardless of whether the assembly is attached to the mask at the time.

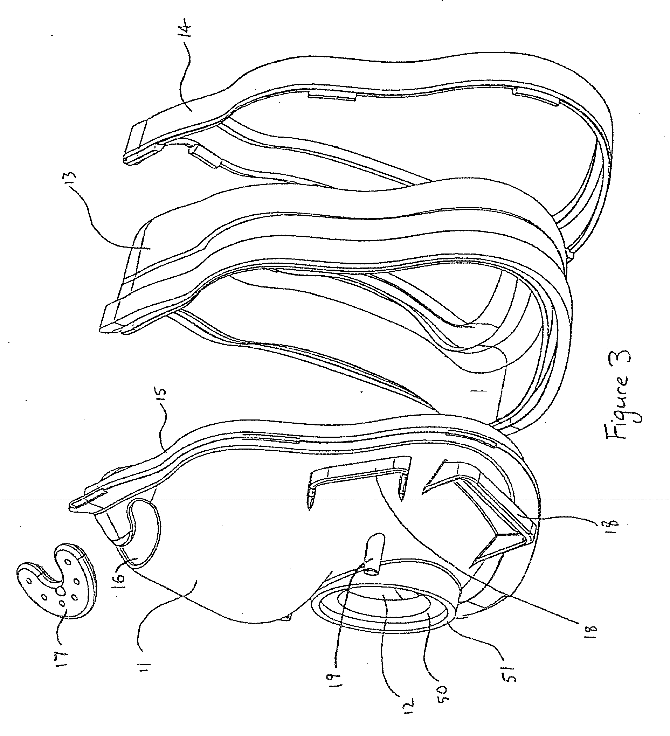

As shown in Figure 3, the mask includes a mask frame 11, cushion 13 and cushion clip 14. The cushion is received on a rib 15 extending around the periphery of the mask frame 11. The cushion is held to the rib by the cushion clip 14. The mask frame includes attachment points 18 that receive straps (not shown) for attaching the mask to the patient, an aperture 16 for receiving an air vent 17, and measurement ports 19.

While particular embodiments of this invention have been described, it will be evident to those skilled in the art that the present invention may be embodied in other specific forms without departing from the essential characteristics thereof. The present embodiments and examples are therefore to be considered in all respects as illustrative and not restrictive, the scope of the invention being indicated by the appended claims rather than the foregoing description, and all changes which come within the meaning and range of equivalency of the claims are therefore intended to be embraced therein.

## Claims

1. A patient gas delivery apparatus including:
a mask adapted for communication with a patient's airways;
a gas flow generator and gas delivery conduit means, further including an assembly connected in series between the conduit and the mask;
said assembly being formed in at least two parts connected by interengaging connecting means, said assembly further including means for connection to the mask, wherein connection of the assembly to the mask prevents incorrect assembly or incorrect alignment of said parts.

2. A patient gas delivery apparatus according to claim 1, wherein the interengaging means connecting the two parts of the assembly includes detent means on a first of the parts which releasably engage a second of the parts, the detents being held in an engaged position by the mask whilst the assembly is connected to the mask.

3. Patient gas delivery apparatus according to claim 2 wherein said first and second parts form a housing for a flow sensor of said apparatus.

4. Patient gas delivery apparatus according to claim 2 wherein said first and second parts form a housing for an anti-asphyxia valve member.

5. Patient gas delivery apparatus according to claim 4 wherein said housing has at least one vent which is closed by said valve member during normal operation of said apparatus, opening when pressure falls below a predetermined pressure.

6. Patient gas delivery apparatus according to claim 2 wherein said second part includes said means for connection of the assembly to the mask.

7. Patient gas delivery apparatus according to claim 6 wherein said means for connection to the mask includes a mating portion for insertion into an aperture of the mask and locking means attachable to said mating portion from an inner side of the mask so as to prevent withdrawal of the mating portion from said aperture, said detent means being prevented from disengagement from said second part whilst said mating portion is inserted in said aperture.

8. Patient gas delivery apparatus according to claim 7 wherein said detent means is prevented from said disengagement by contact with the mask.

9. Patient gas delivery apparatus according to claim 8 wherein the detent means is resiliently biased in a radial direction relative to a common axis of said aperture and said mating portion such that the detent means engages behind a respective formation on said second part and wherein opposite radial movement of said detent means to disengage from said second part is prevented by said contact.

10. Patient gas delivery apparatus according to claim 9 wherein said disengagement is prevented by a projection on the mask.

11. Patient gas delivery apparatus according to claim 10 wherein said projection includes a projecting rim surrounding said aperture.

12. Patient gas delivery apparatus according to any one of the preceding claims wherein the connecting means has a collar that abuts a corresponding surface of the mask to limit the distance that the connecting means can be inserted into the mask aperture.

13. Patient gas delivery apparatus according to claim 13 wherein the corresponding surface is an annulus having a protruding rim such that thus preventing the assembly from separating whilst still attached to the mask frame and/or thus preventing the assembly from being disassembled whilst the assembly is connected to the mask.

14. Patient gas delivery apparatus according to any one of the preceding claims wherein the assembly is an elbow joint.

15. Patient gas delivery apparatus according to any one of the preceding claims, the connecting assembly including an anti-asphyxia valve.
